# DEMANDE DE BREVET EUROPEEN

(11) **EP 1 862 150 A1**
(43) Date de publication de la demande: **05.12.2007**
(21) Numéro de dépôt: 07356069.0
(22) Date de dépôt: 21.05.2007
(51) Int. Cl.: A61F 2/38

(54) **Prothèse totale d'articulation du genou**

(30) Priorité: 30.05.2006 FR 0604785
(71) Demandeur: Dedienne Sante, 34130 Mauguio (FR)
(72) Inventeur: Fonfreide, Gérard, 34130 Mauguio (FR)
(74) Mandataire: Jeannet, Olivier

(57) **Abrégé**

Cette prothèse comprend un élément fémoral (100) présentant, entre les condyles (105), une cavité (107) et une paroi cylindrique convexe (109) ; le plateau intermédiaire que la prothèse comprend également présente une nervure médiane saillante dans la partie postérieure de laquelle est aménagée une portée concave en arc de cercle, cette nervure médiane saillante étant destinée à être reçue de manière ajustée dans ladite cavité (107), ladite paroi cylindrique convexe (109) étant destinée à coopérer de manière congruente avec ladite portée concave lors du mouvement de l'élément fémoral (100) par rapport au plateau intermédiaire.

Selon l'invention,
- les arêtes (120) formées par les parois (121) délimitant latéralement ladite cavité (107) et par les condyles (105) sont arrondies selon des rayons allant en augmentant depuis les zones postérieures des condyles (105) vers les zones antérieures de ces mêmes condyles, et
- l'arête (122) formée par la paroi (119) délimitant ladite cavité (107) du côté antérieur et par la paroi (106) formant la zone médiane antérieure de l'élément fémoral (100) est également arrondie.

## Description

La présente invention concerne une prothèse totale d'articulation du genou, destinée à être implantée avec ablation du ligament croisé postérieur.

Cette prothèse est du même type que celle décrite dans la demande de brevet français N° 95 076781 et est représentée sur les figures 1 et 2 annexées.

Comme cela est représenté, cette prothèse comprend un élément fémoral 1, un élément tibial 2 et un plateau intermédiaire 3.

L'élément fémoral 1 présente, au niveau de sa face externe, deux parties saillantes 5 reproduisant les condyles fémoraux, et une partie médiane 6. Cette partie médiane 6 reproduit une trochlée dans la partie antérieure de l'élément 1, comprend une cavité 7 dans sa zone intermédiaire-antérieure, délimitée par une cage intercondylienne 8, et comprend une paroi cylindrique convexe 9 dans sa zone postérieure, occupant en partie la zone postérieure de la cage 8 et s'étendant entre les zones postérieures des condyles 5. Ces condyles 5 ont une courbure en arc de cercle au niveau de leurs parties postérieures, et la paroi cylindrique 9 a un axe confondu avec l'axe du cercle dans lequel sont inscrites ces parties postérieures.

L'élément tibial 2 comprend une paroi supérieure 10, sensiblement plane, destinée à recevoir le plateau 3, qui présente un pion cylindrique médian 11 faisant saillie de sa face supérieure.

Le plateau intermédiaire 3 est en matériau favorisant le glissement, tel que du polyéthylène à haute densité. Il comprend, du côté de l'élément fémoral 1, deux cavités glénoïdes latérales 15 recevant les condyles 5, et, du côté de l'élément tibial 2, une surface sensiblement plane, destinée à venir prendre appui sur la paroi 10. Les cavités glénoïdes 15 présentent des parties postérieures en arc de cercle, ayant sensiblement le même rayon que les parties postérieures des condyles 5, et ayant un axe confondu avec celui de ces mêmes parties postérieures.

En outre, le plateau intermédiaire 3 comprend une nervure médiane saillante 16, dans la face postérieure de laquelle est aménagée une portée concave 17 en arc de cercle. Cette portée a le même rayon, au jeu près, que la paroi cylindrique 9 et a un axe confondu avec celui de cette paroi 9.

En avant de la paroi 9, la nervure médiane 16 présente une face antérieure 18 légèrement arrondie, et la paroi 19 de l'élément fémoral 1 délimitant la cavité 7 du côté antérieur présente une forme correspondante, cette paroi 19 étant prévue pour venir en appui contre cette face 18 lorsque l'articulation est en position d'extension.

La nervure médiane 16 a une largeur constante correspondant, au jeu près, à la largeur de la cavité 7, de sorte que la cage intercondylienne 8 vient coiffer la nervure 16 sans jeu latéral.

Dans sa face inférieure, le plateau intermédiaire 3 comprend une cavité de forme oblongue, dont la longueur est orientée dans la direction antéropostérieure de la prothèse. Cette cavité peut recevoir le pion 11 avec possibilité de pivotement et de coulissement.

La paroi 9 et la portée 17 constituent un véritable "troisième condyle", permettant une postéro-stabilisation de l'articulation, un parfait guidage de l'élément fémoral 1 sur le plateau intermédiaire 3, autour d'un axe transversal précis et selon un mouvement de flexion harmonieux, ainsi que le maintien d'une surface de contact importante quel que soit le degré de flexion de l'articulation.

La prothèse peut également comprendre un implant rotulien mis en place sur la rotule et coopérant avec la trochlée prothétique.

Cette prothèse connue donne satisfaction en pratique. Il s'avère cependant que l'implant rotulien, également en polyéthylène, subit une usure importante. Cette usure n'est pas souhaitable non seulement pour le fonctionnement de la prothèse mais également par le fait qu'elle génère des particules de polyéthylène qui se diffusent dans l'organisme du patient.

De plus, la trochlée de la prothèse existante apparaît ne pas coopérer de manière optimale avec cet implant rotulien.

La présente invention vise à remédier à ces inconvénients.

Le document N° FR 2 852 819 décrit une prothèse de genou d'un autre type que celui précité, ne comprenant pas un élément fémoral ayant une cavité et un troisième condyle en arrière de cette cavité, et ne comprenant pas un élément tibial ayant une nervure médiane saillante formant une portée concave en arc de cercle, destinée à être introduite dans ladite cavité et à recevoir le troisième condyle avec possibilité de pivotement.

La prothèse selon ce document antérieur ne permet pas de remédier aux inconvénients précités d'usure de l'implant rotulien et d'absence de coopération optimale de la trochlée avec l'implant rotulien.

La prothèse concernée par l'invention comprend, de manière connue en soi, un élément destiné à être ancré dans l'extrémité du fémur, reproduisant les condyles fémoraux, un élément destiné à être ancré dans l'extrémité du tibia, qui présente une paroi supérieure sensiblement plane, et un plateau intermédiaire destiné à assurer le glissement de ces éléments l'un par rapport à l'autre ; les condyles prothétiques de l'élément fémoral ont une courbure en arc de cercle au niveau de leurs parties postérieures, et l'élément fémoral présente, entre ces condyles, une cavité et une paroi cylindrique convexe sensiblement coaxiale au cercle dans lequel sont inscrites les parties postérieures des condyles ; le plateau intermédiaire comprend une nervure médiane saillante, dans la partie postérieure de laquelle est aménagée une portée concave en arc de cercle, cette nervure médiane saillante étant destinée à être reçue de manière ajustée dans ladite cavité, ladite paroi cylindrique convexe étant destinée à coopérer de manière congruente avec ladite portée concave lors du mouvement de l'élément fémoral par rapport au plateau intermédiaire ; les cavités glénoïdes du plateau intermédiaire présentent des parties postérieures congruentes aux parties postérieures des condyles ; le plateau intermédiaire et l'élément tibial comprennent des moyens permettant une mobilité multidirectionnelle et en pivotement du plateau intermédiaire par rapport à l'élément tibial.

Selon invention,
- les arêtes formées par les parois délimitant latéralement ladite cavité et par les condyles sont arrondies selon des rayons allant en augmentant depuis les zones postérieures des condyles vers les zones antérieures de ces mêmes condyles, et
- l'arête formée par la paroi délimitant ladite cavité du côté antérieur et par la paroi formant la zone médiane antérieure de l'élément fémoral est également arrondie.

La demanderesse a en effet pu déterminer que l'usure de l'élément rotulien résultait du frottement de cet élément contre les arêtes vives délimitant la cavité homologue dans la prothèse antérieure, mais qu'un arrondissement excessif des arrêtes latérales conduirait à une perte de congruence de l'élément fémoral par rapport au plateau intermédiaire, particulièrement dans les angles de flexion importants.

Dans la prothèse selon l'invention, la zone antérieure des arêtes délimitant latéralement la cavité, et l'arête délimitant cette cavité du côté antérieur, sont suffisamment arrondies pour ne pas générer une usure de l'implant rotulien lorsque cet implant vient porter contre elles, dans les angles importants de flexion de l'articulation ; les arêtes délimitant la cavité latéralement sont par ailleurs faiblement arrondies dans les zones postérieures des condyles, de sorte qu'une stabilité satisfaisante dans la direction médio-latérale est conservée dans ces angles de flexion importants.

À titre indicatif, lesdits rayons peuvent être de l'ordre de 1,5 mm au niveau des zones postérieures des condyles latéraux et de 5 mm au niveau des zones antérieures de ces condyles ; le rayon générant l'arrondi de l'arête formée par la paroi délimitant la cavité du côté antérieur et par la paroi formant la zone médiane antérieure de l'élément fémoral peut être de l'ordre de 10 mm.

La réduction de l'usure de l'implant rotulien permet par ailleurs de concevoir un implant rotulien et une trochlée prothétique assurant un guidage efficace de la rotule par la trochlée. Ainsi, la direction longitudinale de la trochlée prothétique peut être inclinée du côté externe de l'articulation par rapport au plan médian de l'élément fémoral, afin de garantir une course rotulienne optimale en extension. Cette inclinaison peut être de l'ordre de 5 à 10 degrés par rapport audit plan médian.

L'implant rotulien que peut comprendre la prothèse selon l'invention présente avantageusement une partie en forme de dôme dont le sommet peut être décalé sur le côté externe de cet implant. L'implant rotulien est ainsi asymétrique dans la direction médio-latérale afin de garantir une trajectoire optimale le long de la trochlée, lorsque la prothèse est en extension.

La trochlée prothétique peut être fortement creusée, c'est-à-dire avoir une profondeur allant de 4,5 à 5 mm environ. Cette profondeur augmentée de la trochlée prothétique est favorable au bon fonctionnement de l'implant rotulien.

Par ailleurs, les arrondis précités des arêtes délimitant latéralement ladite cavité permettent l'aménagement sur le plateau intermédiaire de zones de transition arrondies, concaves, entre les parois latérales de ladite nervure médiane saillante et les surfaces glénoïdes adjacentes de ce plateau intermédiaire.

Ces zones de transition arrondies permettent de répartir de manière plus favorable les contraintes exercées sur le plateau intermédiaire dans la direction médio-latérale, et donc de réduire le risque d'altération à cet endroit de la matière constituant ce plateau.

L'invention sera bien comprise, et d'autres caractéristiques et avantages de celle-ci apparaîtront, en référence au dessin schématique annexé, représentant, à titre d'exemple non limitatif, une forme de réalisation préférée de l'élément fémoral et de l'élément rotulien de la prothèse qu'elle concerne.

La figure 3 est une vue en perspective de l'élément fémoral ;

la figure 4 en est une vue en perspective, sous un autre angle ;

la figure 5 en est une vue de côté.

les figures 6 à 8 en sont des vues en coupe selon respectivement les lignes VI-VI, VII-VII et VIII-VIII de la figure 5, et

la figure 9 est une vue de côté de l'élément rotulien.

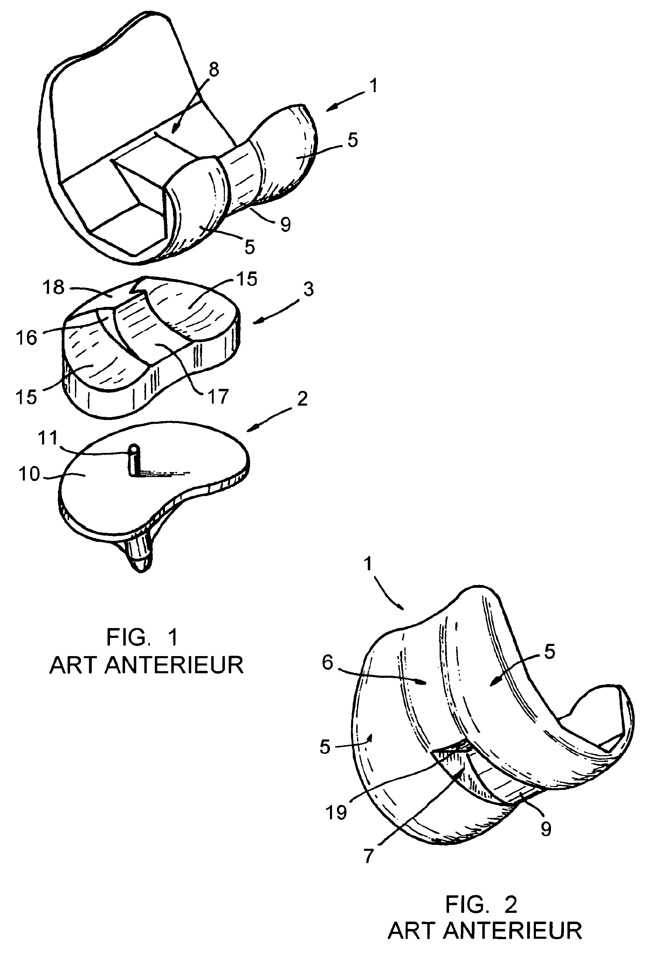

Comme le montrent les figures 1 à 8, l'élément fémoral 100 selon l'invention présente, à l'instar de l'élément fémoral 1 connu, deux parties saillantes latérales 105 reproduisant les condyles fémoraux, et une partie médiane 106. Cette partie médiane 106 reproduit une trochlée dans la partie antérieure de l'élément 100, comprend une cavité 107 dans sa zone intermédiaire-antérieure, délimitée par une cage intercondylienne 108, et comprend une paroi cylindrique convexe 109 dans sa zone postérieure, occupant en partie la zone postérieure de la cage 108 et s'étendant entre les zones postérieures des condyles 105. Ces condyles 105 ont une courbure en arc de cercle au niveau de leurs parties postérieures, et la paroi cylindrique 109 a un axe confondu avec l'axe du cercle dans lequel sont inscrites ces parties postérieures.

Sur l'élément fémoral 100,
- les arêtes 120 formées par les parois 121 délimitant latéralement la cavité 107 et par les condyles 105 sont arrondies selon des rayons allant en augmentant depuis les zones postérieures des condyles 105 vers les zones antérieures de ces mêmes condyles, et
- l'arête 122 formée par la paroi 119 délimitant la cavité 107 du côté antérieur et par la paroi formant la zone médiane antérieure 106 de l'élément fémoral 100 est également arrondie.

Ainsi, dans la prothèse selon l'invention, la zone antérieure des arêtes 120 et l'arête 122 sont suffisamment arrondies pour ne pas générer une usure de l'implant rotulien 130 lorsque cet implant 130 vient porter contre elles, dans les angles importants de flexion de l'articulation ; les arêtes 120 sont par ailleurs faiblement arrondies dans les zones postérieures des condyles 105, de sorte qu'une stabilité satisfaisante dans la direction médio-latérale est conservée dans ces mêmes angles de flexion importants.

Les rayons des arrondis des arêtes 120 sont de l'ordre de 1,5 mm au niveau des zones postérieures des condyles 105 et de 5 mm au niveau des zones antérieures de ces condyles 105 ; le rayon générant l'arrondi de l'arête 122 est de l'ordre de 10 mm.

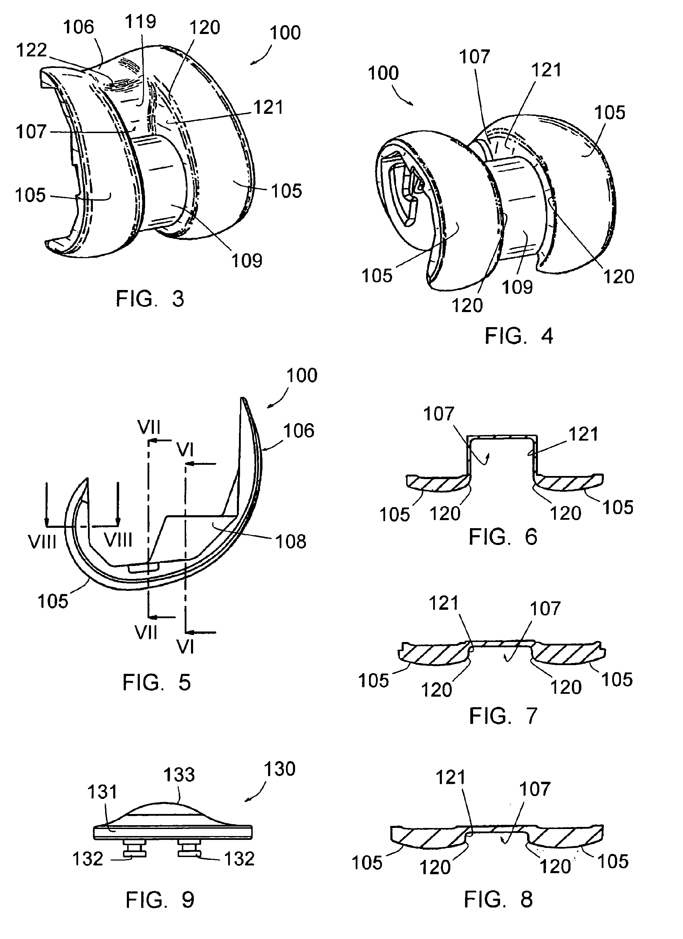

Comme le montre la figure 9, l'implant rotulien 130 comprend une partie de base plane 131 destinée à venir porter contre l'os de la rotule dûment réséqué, deux plots 132 d'ancrage dans cet os et une partie 133 en forme de dôme. Le sommet de cette partie 133 est décalé sur le côté externe de l'implant 130. L'implant rotulien 130 est ainsi asymétrique dans la direction médio-latérale afin de garantir une trajectoire optimale le long de la trochlée lorsque la prothèse est en extension.

La direction longitudinale de la trochlée de l'élément fémoral 100 est inclinée du côté externe de l'articulation par rapport au plan médian de l'élément fémoral, formant un angle de l'ordre de 5 à 10 degrés par rapport audit plan médian, et cette trochlée est fortement creusée, ayant une profondeur de 4,5 à 5 mm.

L'élément tibial et le plateau intermédiaire de la prothèse selon l'invention peuvent être identiques à ceux décrits en référence aux figures 1 et 2. Le plateau intermédiaire peut comprendre des zones de transition arrondies entre les parois latérales de la nervure médiane saillante et les surfaces glénoïdes adjacentes. Ces zones arrondies, concaves, sont rendues possibles du fait de l'existence des arrondis des arêtes 120.

Comme cela apparaît de ce qui précède, l'invention fournit une prothèse totale d'articulation du genou, destinée à être implantée avec ablation du ligament croisé postérieur, qui présente les avantages déterminants d'avoir un implant rotulien subissant une usure faible, de pouvoir comprendre un implant rotulien et une trochlée coopérant de manière optimale l'un avec l'autre, et de permettre l'aménagement desdites zones de transition arrondies, ces zones permettant de répartir de manière plus favorable les contraintes exercées sur le plateau intermédiaire dans la direction médio-latérale, et donc de réduire le risque d'altération à cet endroit de la matière constituant ce plateau.

Il va de soi que l'invention n'est pas limitée à la forme de réalisation décrite ci-dessus à titre d'exemple mais qu'elle s'étend à toutes les formes de réalisation couvertes par les revendications ci-annexées.

## Revendications

1. - Prothèse totale d'articulation du genou comprenant un élément (100) destiné à être ancré dans l'extrémité du fémur, reproduisant les condyles fémoraux, un élément (2) destiné à être ancré dans l'extrémité du tibia, qui présente une paroi supérieure (10) sensiblement plane, et un plateau intermédiaire (2) destiné à assurer le glissement de ces éléments (100, 2) l'un par rapport à l'autre ; les condyles prothétiques (105) de l'élément fémoral (100) ont une courbure en arc de cercle au niveau de leurs parties postérieures, et l'élément fémoral (100) présente, entre ces condyles (105), une cavité (107) et une paroi cylindrique convexe (109) sensiblement coaxiale au cercle dans lequel sont inscrites les parties postérieures des condyles (105) ; le plateau intermédiaire (3) comprend une nervure médiane saillante (16), dans la partie postérieure de laquelle est aménagée une portée concave (17) en arc de cercle, cette nervure médiane saillante (16) étant destinée à être reçue de manière ajustée dans ladite cavité (107), ladite paroi cylindrique convexe (109) étant destinée à coopérer de manière congruente avec ladite portée concave (17) lors du mouvement de l'élément fémoral (100) par rapport au plateau intermédiaire (3) ; les cavités glénoïdes (15) du plateau intermédiaire (3) présentent des parties postérieures congruentes aux parties postérieures des condyles (105) ; le plateau intermédiaire (3) et l'élément tibial (2) comprennent des moyens (11) permettant une mobilité multidirectionnelle et en pivotement du plateau intermédiaire (3) par rapport à l'élément tibial (2) ;
prothèse **caractérisée en ce que** :
- les arêtes (120) formées par les parois (121) délimitant latéralement ladite cavité (107) et par les condyles (105) sont arrondies selon des rayons allant en augmentant depuis les zones postérieures des condyles (105) vers les zones antérieures de ces mêmes condyles, et
- l'arête (122) formée par la paroi (119) délimitant ladite cavité (107) du côté antérieur et par la paroi (106) formant la zone médiane antérieure de l'élément fémoral (100) est également arrondie.

2. - Prothèse selon la revendication 1, **caractérisée en ce que** lesdits rayons sont de l'ordre de 1,5 mm au niveau des zones postérieures des condyles (105) et de 5 mm au niveau des zones antérieures de ces condyles (105).

3. - Prothèse selon la revendication 1 ou la revendication 2, **caractérisée en ce que** le rayon générant l'arrondi de l'arête (122) formée par la paroi (119) délimitant la cavité (107) du côté antérieur et par la paroi (106) formant la zone médiane antérieure de l'élément fémoral (100) est de l'ordre de 10 mm.

4. - Prothèse selon l'une des revendications 1 à 3, **caractérisée en ce que** la direction longitudinale de la trochlée prothétique est inclinée du côté externe de l'articulation par rapport au plan médian de l'élément fémoral (100).

5. - Prothèse selon la revendication 4, **caractérisée en ce que** l'inclinaison de la direction longitudinale de la trochlée est de l'ordre de 5 à 10 degrés par rapport au plan médian de l'élément fémoral (100).

6. - Prothèse selon l'une des revendications 1 à 5, **caractérisée en ce qu'**elle comprend un implant rotulien (130) présentant une partie (133) en forme de dôme dont le sommet est décalé sur le côté externe de cet implant (130).

7. - Prothèse selon l'une des revendications 1 à 6, **caractérisée en ce que** la trochlée prothétique est fortement creusée, c'est-à-dire a une profondeur allant de 4,5 à 5 mm environ.

8. - Prothèse selon l'une des revendications 1 à 7, **caractérisée en ce que** le plateau intermédiaire comprend des zones de transition arrondies, concaves, entre les parois latérales de ladite nervure médiane saillante et les surfaces glénoïdes adjacentes.
